## Europäisches Patentamt
## European Patent Office
## Office européen des brevets

(11) Veröffentlichungsnummer: **0 273 275 B1**

(12) **EUROPÄISCHE PATENTSCHRIFT**

(45) Veröffentlichungstag der Patentschrift: **18.12.91**

(51) Int. Cl.⁵: **G01N 31/12**

(21) Anmeldenummer: **87118404.0**

(22) Anmeldetag: **11.12.87**

(54) **Crack-reaktor.**

(30) Priorität: **23.12.86 DE 3644277**

(43) Veröffentlichungstag der Anmeldung:
**06.07.88 Patentblatt 88/27**

(45) Bekanntmachung des Hinweises auf die
Patenterteilung:
**18.12.91 Patentblatt 91/51**

(84) Benannte Vertragsstaaten:
**DE FR GB IT**

(56) Entgegenhaltungen:
**US-A- 3 084 031**
**US-A- 4 568 426**
**US-A- 4 601 882**

(73) Patentinhaber: **SIEMENS AKTIENGESELL-
SCHAFT**
**Wittelsbacherplatz 2**
**W-8000 München 2(DE)**

(72) Erfinder: **Leonhardt, Dietrich**
**Bulacher Strasse 32**
**W-7505 Ettlingen(DE)**

EP 0 273 275 B1

## Beschreibung

Die Erfindung bezieht sich auf einen Crack-Reaktor für die Bestimmung sauerstoffhaltiger Komponenten eines Gasgemisches (Probengas), im wesentlichen bestehend aus einer vom Probengas durchströmten, auf Temperaturen über 1500 K direkt aufheizbaren Metallkapillare.

Bei bekannten Ausführungen ist die Metallkapillare, die vorzugsweise aus Platin oder einer Platinlegierung besteht, auf einen keramischen Trägerkörper als Wendel aufgewickelt und von einer Isoliermasse umgeben. Die Metallkapillare ist an eine Stromquelle angeschlossen und direkt auf eine Temperatur von etwa 1500 K aufheizbar. In dem Crack-Reaktor wird aus zugeführten sauerstoffhaltigen organischen Verbindungen Kohlenoxyd gebildet. In den bekannten Systemen zur selektiven gaschromatographischen Messung sauerstoffhaltiger Verbindungen mittels Flammenionisationsdetektor wird der Crack-Reaktor einer Kapillartrennsäule unmittelbar nachgeschaltet. Das bei der Aufspaltung sauerstoffhaltiger organischer Verbindungen in dem Crack-Reaktor entstehende Kohlenoxyd wird in einem folgenden Hydrier-Reaktor unter Zugabe von Wasserstoff zu Methan hydriert, welches mit einem Flammmenionisationsdetektor in bekannter Weise gemessen und angezeigt wird.

Es hat sich herausgestellt, daß bei den benötigten hohen Temperaturen die Standzeit der Metallkapillaren wegen auftretender Porosität relativ gering ist.

Es besteht demgemäß die Aufgabe, die Standzeit der Metallkapillare und damit die Betriebsdauer des Crack-Reaktors zu verlängern.

Eine Lösung der Aufgabe wird darin gesehen, daß die Metallkapillare im Crack-Reaktor in einer sauerstofffreien Gasatmosphäre betrieben wird.

Diese sauerstofffreie Gasatmosphäre kann aus einem Edelgas, beispielsweise Argon, oder einem Edelgasgemisch bestehen, welches sich entweder in einem die gewendelte Metallkapillare umgebenden, hermetisch abgeschlossenen Raum befindet oder einen die Metallkapillare umgebenden Raum durchströmt.

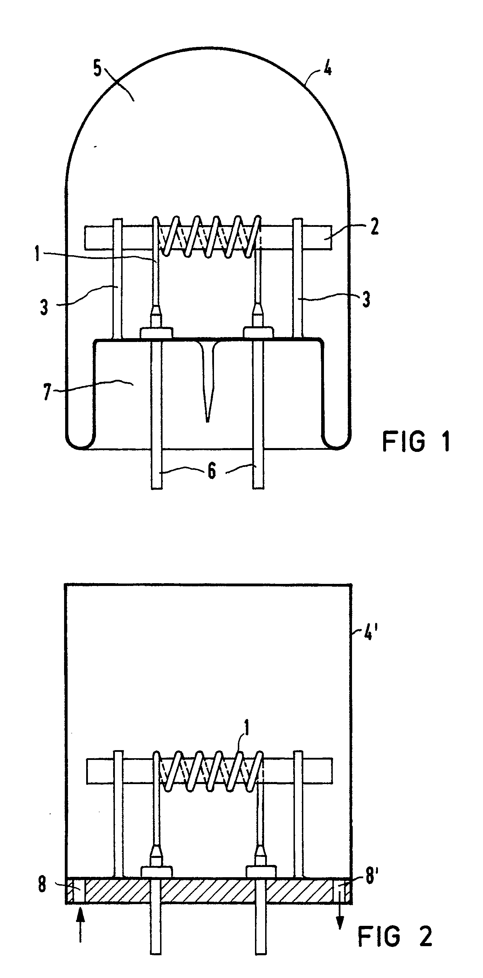

Mit den Figuren 1 und 2 sind zwei Ausführungsbeispiele eines erfindungsgemäßen Crack-Reaktors schematisch dargestellt und im folgenden beschrieben.

Figur 1: Die aus einer Pt/Rh-Legierung bestehende Metallkapillare 1 ist als Wendel auf einen stabförmigen Keramikkörper 2 aufgewickelt, der von zwei Stützen 3 gehalten wird. Diese Anordnung befindet sich in einem hermetisch geschlossenen Gehäuse 4, vorzugsweise aus Glas, welches mit einer sauerstofffreien Gasatmosphäre 5, z. B. mit Argon, gefüllt ist. Die Enden der Metallkapillare 1 sind mit den der Zu- und -abführung des Probengases dienenden Anschlußstutzen 6 verbunden, die durch den Hartglassockel 7 nach außen geführt sind. Die Zuführung des Heizstroms von ca. 20 A zu der gewendelten Metallkapillare erfolgt ebenfalls über die metallenen Anschlußstutzen 6. Bei der Betriebstemperatur von etwa 1500 K herrscht in dem hermetisch abgeschlossenen Raum ein leichter Überdruck.

Eine andere Ausführungsform zeigt Figur 2. Die Metallkapillare 1 des Crack-Reaktors ist hier in einem Gehäuse 4' angeordnet, welches Anschlüsse 8, 8' aufweist für die Zu- bzw. Abfuhr eines sauerstofffreien Gases oder Gasgemisches, welches den die Metallkapillare 1 umgebenden Raum durchströmt.

## Patentansprüche

1. Crack-Reaktor für die Bestimmung sauerstoffhaltiger Komponenten eines Gasgemisches (Probengas), im wesentlichen bestehend aus einer vom Probengas durchströmten, auf Temperaturen über 1500 K direkt aufheizbaren Metallkapillare, **dadurch gekennzeichnet, daß** die Metallkapillare (1) von einer sauerstofffreien Gasatmosphäre umgeben ist.

2. Crack-Reaktor nach Anspruch 1, **dadurch gekennzeichnet, daß** die Metallkapillare (1) in einem mit sauerstofffreiem Gas (5) gefüllten Raum hermetisch eingeschlossen ist.

3. Crack-Reaktor nach Anspruch 1, **dadurch gekennzeichnet, daß** die Metallkapillare (1) in einer von sauerstofffreiem Gas durchströmten Gehäuse (4') angeordnet ist.

4. Crack-Reaktor nach einem der Ansprüche 1, 2 oder 3, **dadurch gekennzeichnet, daß** das sauerstofffreie Gas ein Edelgas oder Edelgasgemisch ist.

## Claims

1. Cracking reactor for the determination of oxygen-containing components of a gas mixture (test gas), substantially consisting of a metal capillary tube, through which the test gas flows and which can be heated directly to temperatures over 1500 K, characterized in that the metal capillary tube (1) is surrounded by an oxygen-free gas atmosphere.

2. Cracking reactor according to claim 1, characterized in that the metal capillary tube (1) is hermetically enclosed in a space filled with oxygen-free gas (5).

3.  Cracking reactor according to claim 1, characterized in that the metal capillary tube (1) is arranged in a housing (4') through which oxygen-free gas flows.

4.  Cracking reactor according to one of claims 1, 2 or 3, characterized in that the oxygen-free gas is a noble gas or a noble-gas mixture.

## Revendications

1.  Réacteur de craquage pour la détermination des constituants oxygénés d'un mélange gazeux (gaz-échantillon), constitué essentiellement d'un tube capillaire métallique parcouru par le gaz-échantillon et pouvant être chauffé directement à des températures supérieures à $1500\,^{\circ}$ K, caractérisé en ce que le tube capillaire métallique (1) est entouré d'une atmosphère gazeuse exempte d'oxygène.

2.  Réacteur de craquage suivant la revendication 1, caractérisé en ce que le tube capillaire métallique (1) est enfermé hermétiquement dans une chambre emplie de gaz (5) exempt d'oxygène.

3.  Réacteur de craquage suivant la revendication 1, caractérisé en ce que le tube capillaire métallique (1) est disposé dans une enveloppe (4') parcourue par du gaz exempt d'oxygène.

4.  Réacteur de craquage suivant l'une des revendications 1, 2 ou 3, caractérisé en ce que le gaz exempt d'oxygène est un gaz rare ou un mélange de gaz rares.

EP 0 273 275 B1

FIG 1

FIG 2